# EUROPEAN PATENT APPLICATION

(11) **EP 4 617 363 A1**
(43) Date of publication of application: **17.09.2025**
(21) Application number: 25157880.3
(22) Date of filing: 14.02.2025
(51) Int. Cl.: C12N 5/0793

(54) **AMYOTROPHIC LATERAL SCLEROSIS (ALS) MODEL CELL, PRODUCTION METHOD FOR ALS MODEL CELLS, AND SCREENING METHOD FOR PROPHYLACTIC OR THERAPEUTIC DRUG FOR ALS**

(30) Priority: 15.03.2024 JP 2024041461; 30.10.2024 JP 2024190695
(71) Applicant: Ricoh Company, Ltd., Tokyo 143-8555 (JP)
(72) Inventor: IJICHI, Rie, Tokyo, 143-8555 (JP)
(74) Representative: Marks & Clerk LLP

(57) **Abstract**

What is provided is an ALS model cell that can be conveniently produced and is associated with a high proportion of cells in which a disease condition phenotype of ALS is reproduced. Disclosed are: a nerve cell having a causative gene of ALS incorporated into the genome of the nerve cell in an acquired manner; a co-culture product containing the nerve cell and an astrocyte having a causative gene of ALS incorporated into the genome of the astrocyte; a production method for ALS model cells, the production method including a step of introducing a causative gene of ALS operably linked to a promoter for a eukaryotic cell, into a genome of nerve cells or into a genome of a co-culture product of nerve cells and astrocytes; and a screening method for a prophylactic or therapeutic drug for ALS, the screening method including: a step of culturing ALS model cells in presence of a test substance; and a step of evaluating a phenotype of the ALS model cells, in which the phenotype of the ALS model cells being closer to a wild-type phenotype as compared with a phenotype in absence of the test substance, implies that the test substance is a prophylactic or therapeutic drug for ALS.

## Description

### BACKGROUND OF THE INVENTION

### Field of the Invention

The present invention relates to an amyotrophic lateral sclerosis (ALS) model cell, a production method for ALS model cells, and a screening method for a prophylactic or therapeutic drug for ALS. The present application claims priority on Japanese Patent Application No. 2024-041461, filed on March 15, 2024, and Japanese Patent Application No. 2024-190695, filed on October 30, 2024, the contents of which are incorporated herein by references.

### Description of Related Art

In vitro disease models using human cells have been applied to research for elucidation of disease mechanisms and drug discovery. In particular, disease models using human induced pluripotent stem cell (iPSC)-derived differentiated cells are expected as in vitro disease models with high reproducibility because the cells are more easily obtained as compared to human primary cultured cells, and there is less variation between lots.

When a genetic mutation that causes a disease has been identified, and a patient has the genetic mutation, iPSCs are established from patient-derived cells and then induced to differentiate into cells that constitute a tissue that is a target of the disease, and in a case where a disease-specific phenotype is observed, the iPSCs are utilized as a disease model. However, because iPSCs derived from healthy individuals and iPSCs derived from diseases have different genetic backgrounds other than the disease-causative gene, it may be difficult to evaluate disease-specific phenotypes depending on the disease, and even in a case where a disease-specific phenotype is recognized, it may be difficult to conclude whether it is due to the causative gene.

In this regard, studies have been conducted to produce a disease model by obtaining disease model iPSCs by introducing a disease-causative gene into iPSCs established from cells derived from healthy individuals, knocking down the disease-causative gene, or subjecting the disease-causative gene to genome editing, and inducing differentiation of the iPSCs.

Furthermore, control strains in which a disease-causative gene of iPSCs established from patient-derived cells has been repaired by genome editing, have also been produced.

When these methods are used, the genetic background can be made uniform, and therefore, it is possible to conduct more rigorous evaluation of disease-specific phenotypes.

Meanwhile, amyotrophic lateral sclerosis (ALS) is a progressive neurodegenerative disease in which motor neurons are damaged. Twenty or more causative genes have been revealed so far; however, the relationship with the disease state and the rate of progression has not been sufficiently clarified, and the detailed mechanism is unknown. No fundamental treatment method has yet been established, and the development of new drugs and new treatment methods is desired.

In experiments using a nerve cell induced to be differentiated from iPSCs derived from ALS patients who have been proved to have a causative gene, and the like, phenotypes such as cell death, a change in the localization of a causative protein, and formation of aggregates are recognized, and it has been considered to utilize the iPSCs as an ALS disease model.

For example, in Oiwa K., et al., "Monomerization of TDP-43 is a key determinant for inducing TDP-43 pathology in amyotrophic lateral sclerosis", Sci Adv., 9, eadf6895, 2023, focusing on dimers and multimers of TDP-43 detected in the postmortem brain and cerebrospinal fluid of ALS patients, an ALS model produced by forcibly expressing TDP-43, which is obtained by introducing a mutation into an interface portion that interacts during the formation of multimers, in nerve cells using a lentivirus, is described. In this ALS model, it is shown that the forced expression of TDP-43 causes a change in the localization of TDP-43, cell death, and cell damage, and experimental results suggest that the disease state of ALS is caused by monomerization of TDP-43.

Furthermore, in Vogt M.A., et al., "TDP-43 induces p53-mediated cell death of cortical progenitors and immature neurons", Sci Rep., 8, 8097, 2018, an ALS model produced by transiently overexpressing a wild type or A315T mutant of TDP-43, which is one of the ALS causing proteins, in iPSC-derived cortical progenitor cells and neurons, is described. In this ALS model, the evaluation is performed two days after the transfection. As a result, it is described that since cell death occurred due to forced expression of TDP-43, and cell death and an increase in the number of Caspase-3-positive cells were suppressed by the addition of a p53 inhibitor, p53-mediated apoptosis is involved in cell death caused by TDP-43.

### SUMMARY OF THE INVENTION

### Technical Problem

However, the mutant of TDP-43 used in Oiwa K., et al. (2023) is not a mutant that is recognized in ALS patients. Furthermore, in the ALS model described in Vogt M.A., et al. (2018), only short-term evaluation can be carried out, and it has not been verified whether the intracellular localization of TDP-43 exhibits the disease condition phenotype of ALS. In the related art, there has been no report of reproducing the disease condition phenotype of ALS in an ALS model in which a causative gene recognized in ALS patients is expressed in iPSC-derived nerve cells.

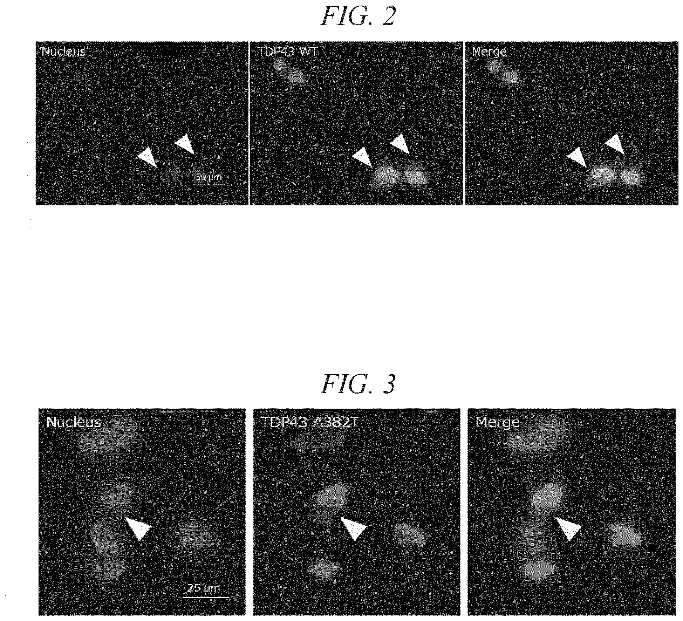

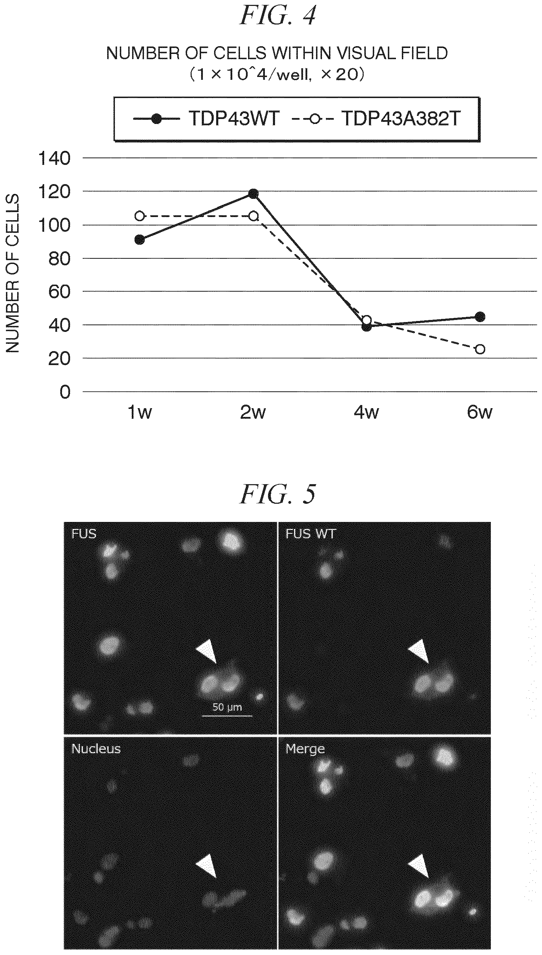

In addition, as shown in Zhang Z., et al., "Downregulation of MicroRNA-9 in iPSC-Derived Neurons of FTD/ALS Patients with TDP-43 Mutations", PLOS ONE, 8, 10, e76055, 2013, and Fujimori K., et al., "Modeling sporadic ALS in iPSC-derived motor neurons identifies a potential therapeutic agent", Nature Medicine, 24, 1579-1589, 2018, in ALS models that use patient-derived iPSC-derived differentiated cells, the proportion of cells exhibiting the disease condition phenotype of ALS may be low. FIG. 4C of Zhang Z., et al. (2013) shows that the proportion in which a change in the localization of TDP-43 from the nucleus to the cytoplasm is observed is about 15%. Furthermore, FIG. 2i of Fujimori K., et al. (2018) shows that the proportion in which a change in the localization of FUS from the nucleus to the cytoplasm is observed is about 30% at the maximum.

The ALS models described in Zhang Z., et al. (2013) and Fujimori K., et al. (2018) have room for improvement in carrying out drug screening using the disease condition phenotype of ALS as an indicator. It is difficult to detect and evaluate the improvement of the disease condition phenotype in a small number of cells using an ALS model including a large number of cells that do not show the disease condition phenotype of ALS, and it cannot be said that the evaluation system is a highly sensitive evaluation system.

An object of the present invention is to provide an ALS model cell that can be conveniently produced and is associated with a high proportion of cells in which the disease condition phenotype of ALS is reproduced.

### Solution to Problem

A nerve cell according to the present invention has a causative gene of ALS that is operably linked to a promoter for a eukaryotic cell, incorporated into a genome in an acquired manner.

### Advantageous Effects of Invention

According to the present invention, an ALS model cell that can be conveniently produced and is associated with a high proportion of cells in which the disease condition phenotype of ALS is reproduced, can be provided.

### BRIEF DESCRIPTION OF THE DRAWINGS

FIG. 1 is a schematic view showing an embodiment of a production method for ALS model cells.
FIG. 2 is fluorescence microscopic images showing results of immunochemical staining in Experimental Example 1.
FIG. 3 is fluorescence microscopic images showing results of immunochemical staining in Experimental Example 2.
FIG. 4 is a graph showing results of measuring changes over time in the number of cells of nerve cells after gene transfer in Experimental Example 4.
FIG. 5 is fluorescence microscopic images showing results of immunochemical staining in Experimental Example 5.
FIG. 6 is fluorescence microscopic images showing results of immunochemical staining in Experimental Example 6.
FIG. 7 is fluorescence microscopic images showing results of immunochemical staining in Experimental Example 7.
FIG. 8 shows fluorescence microscopic images showing results of immunochemical staining in Experimental Example 8.

### DETAILED DESCRIPTION OF THE INVENTION

### [ALS Model]

### (Nerve cell)

According to an embodiment, the present invention provides a nerve cell having a causative gene of ALS, which is operably linked to a promoter for a eukaryotic cell, incorporated into a genome thereof in an acquired manner.

As will be described later in Examples, the nerve cell of the present embodiment can be conveniently produced and allows the disease condition phenotype of ALS to be reproduced therein. In addition, the nerve cell of the present embodiment is associated with a high proportion of cells in which the disease condition phenotype of ALS is reproduced. More specifically, 15% or more, for example, 20% or more, 25% or more, or 30% or more, of the nerve cells of the present embodiment exhibit the disease condition phenotype of ALS.

Therefore, the nerve cell of the present embodiment can be suitably used as an ALS model. Here, the term "ALS model" means a system in which the pathological phenotype of ALS is reproduced, and this includes a cell in which the pathological phenotype of ALS is reproduced and a cell culture product containing the cell; however, in the present disclosure, unless otherwise specified, the "ALS model" is synonymous with "ALS model cell". Here, the disease condition phenotype of ALS is a phenotype that is actually observed in ALS patients, and the details thereof will be described later.

A promoter for a eukaryotic cell is a promoter capable of forcibly expressing a downstream gene in a eukaryotic cell. The gene operably linked to the promoter for a eukaryotic cell is forcibly expressed in the cell. As the eukaryotic cell, an animal cell is preferred. Examples of the animal include primates such as humans and monkeys, mammals such as rabbits and dogs, ungulates such as pigs, sheep, horses, and cows, and rodents such as mice, rats, guinea pigs, and hamsters. More specific examples of the promoter for a eukaryotic cell include a cytomegalovirus (CMV) promoter, a peptide elongation factor 1α (EF-1α) promoter, a simian virus 40 (SV40) promoter, a chicken β-actin (CAG) promoter to which a CMV early enhancer has been added, and a human synapsin 1 (SYN1) promoter.

It is preferable that the nerve cell of the present embodiment is a nerve cell that has been induced to be differentiated from a pluripotent stem cell derived from a healthy individual. As a result, since this makes the genetic background other than the causative gene of ALS comparable to that of a healthy individual, and therefore, it can be determined that the disease condition phenotype of ALS observed in the nerve cell of the present embodiment is a phenotype attributable to the causative gene of ALS.

Examples of the pluripotent stem cell include an embryonic stem cell (ESC), an induced pluripotent stem cell (iPSC), and a nuclear transfer embryonic stem cell (ntESC). Among these, iPSC is preferable as the pluripotent stem cell.

The induction of differentiation of a pluripotent stem cell into a nerve cell can be carried out by, for example, introducing a specific transcription factor into the pluripotent stem cell. The cell obtained by introducing a specific transcription factor into a pluripotent stem cell may be a commercially available cell, and for example, Excitatory Neurons (Elixirgen Scientific, Inc.) can be used. This cell is a pluripotent stem cell that has been subjected to a differentiation induction treatment into a nerve cell, and differentiates into a functionally mature nerve cell in about 10 days.

Examples of the causative gene of ALS include TAR DNA binding protein-43 (TDP-43) gene, Fused in sarcoma (FUS) gene, and Superoxide dismutase 1 (SOD1) gene, all including both wild-type and mutant genes, and preferred examples thereof include wild-type or mutant TDP-43 gene and wild-type or mutant FUS gene. It is known that even a wild-type gene may exhibit the disease condition phenotype of ALS by forced expression.

The NCBI accession number of the cDNA of human TDP-43 gene is NM_007375.4. The NCBI accession numbers of the cDNAs of human FUS gene are NM_001010850.1, NM_001170634.1, NM_001170937.1, NM_004960.4, and the like. The NCBI accession number of the cDNA of human SOD1 gene is NM_000454.5.

Examples of a mutant TDP-43 gene that is known as a causative gene of ALS include genes encoding mutant TDP-43 proteins (G298S, A315T, M337V, Q343R, G348C, N352S, A382T, and the like).

Examples of a mutant FUS gene that is known as a causative gene of ALS include genes encoding mutant FUS proteins (R521L, R521G, R521H, R521C, P525L, R495X, and the like).

Examples of a mutant SOD1 gene that is known as a causative gene of ALS include genes encoding mutant SOD1 proteins (K3E, A4V, G37R, G41S, N86S, D90A, N139D, L144S, and the like).

When it is said that a causative gene of ALS operably linked to a promoter for a eukaryotic cell is incorporated into the genome, it is implied that the causative gene of ALS operably linked to a promoter for a eukaryotic cell is incorporated into the genome of a nerve cell. This allows the causative gene of ALS to be stably expressed for a long period of time. For this reason, it may be possible to express and observe a disease condition phenotype of ALS that is not expressed by short-term expression of the causative gene of ALS.

As will be described later, the causative gene of ALS operably linked to a promoter for a eukaryotic cell can be conveniently incorporated into the genome by introducing the gene into a nerve cell using a lentiviral vector.

Examples of the disease condition phenotype of ALS that can be observed in the nerve cell of the present embodiment include localization of the TDP-43 protein, which is normally localized in the nucleus, in the cytoplasm; aggregation of the TDP-43 protein; localization of the FUS protein, which is normally localized in the nucleus, in the cytoplasm; aggregation of the FUS protein; a decrease in the number of nerve cells (cell death) after the lapse of a long period of time (for example, 4 weeks) from the expression of the causative gene of ALS; retraction of neurites; and formation of stress granules.

### (Co-culture product containing nerve cells and astrocytes)

The nerve cell of the present embodiment may be co-cultured with astrocytes. That is, according to an embodiment, the present invention provides a co-culture product including the above-mentioned nerve cells and astrocytes in which a causative gene of ALS operably linked to a promoter for a eukaryotic cell is incorporated into the genome.

As will be described later in Examples, the co-culture product of the present embodiment can be conveniently produced, and the disease condition phenotype of ALS can be reproduced therein. Therefore, the co-culture product of the present embodiment can also be suitably used as an ALS model. Furthermore, when the nerve cells and the astrocytes are co-cultured, a phenotype closer to that observed in vivo may be observed. The disease condition phenotype of ALS is the same as described above.

In the co-culture product of the present embodiment, the astrocytes may be primary cultured cells or may be cells that have been induced to be differentiated from pluripotent stem cells such as iPSCs. The induction of differentiation of pluripotent stem cells into astrocytes can be carried out by, for example, introducing a specific transcription factor into the pluripotent stem cells. The cells obtained by introducing a specific transcription factor into pluripotent stem cells may be commercially available cells.

In the co-culture product of the present embodiment, the terms "promoter for a eukaryotic cell", "causative gene of ALS", "incorporated into the genome", and the like are the same as described above.

In the co-culture product of the present embodiment, the causative gene of ALS incorporated into the genome of the astrocytes may be the same gene as the causative gene of ALS incorporated into the genome of the nerve cell. As will be described later, such a co-culture product can be conveniently produced by simultaneously introducing a causative gene of ALS into a co-culture product of the nerve cells and the astrocytes.

### [Production method for ALS model cells]

According to an embodiment, the present invention provides a production method for ALS model cells, the method including a step of introducing a causative gene of ALS operably linked to a promoter for a eukaryotic cell, into a genome of nerve cells that have been induced to be differentiated from pluripotent stem cells, or into a genome of a co-culture product of the nerve cells and astrocytes, in which the nerve cells or the co-culture product of the nerve cells and the astrocytes, into which the causative gene of ALS has been incorporated into the genome, are ALS model cells.

The above-described nerve cells or the above-described co-culture product of nerve cells and astrocytes can be produced by the production method of the present embodiment.

In the production method of the present embodiment, instead of introducing the causative gene of ALS into pluripotent stem cells, the causative gene of ALS is introduced into nerve cells after being induced to be differentiated from pluripotent stem cells, or into a co-culture product of nerve cells and astrocytes. For this reason, it may be possible to eliminate the effort of inducing differentiation of pluripotent stem cells after gene transfer into nerve cells or astrocytes and produce an ALS model more conveniently.

In the production method of the present embodiment, it is preferable that the nerve cells are nerve cells that have been induced to be differentiated from pluripotent stem cells derived from a healthy individual. As described above, since this makes the genetic background other than the causative gene of ALS comparable to that of a healthy individual, it can be determined that the disease condition phenotype of ALS observed in an ALS model obtained by the production method according to the present embodiment is a phenotype attributable to the causative gene of ALS.

In the production method of the present embodiment, the causative gene of ALS operably linked to a promoter for a eukaryotic cell is the same as described above. As described above, examples of the causative gene of ALS include TDP-43 gene, FUS gene, and SOD1 gene, all including both wild-type and mutant genes, and preferred examples thereof include wild-type or mutant TDP-43 gene and wild-type or mutant FUS gene.

In the production method of the present embodiment, it is preferable that the step of introducing the causative gene of ALS is carried out using a lentiviral vector.

Lentivirus is a kind of retrovirus, and human immunodeficiency virus (HIV), simian immunodeficiency virus (SIV), feline immunodeficiency virus (FIV), equine infectious anemia virus, bovine immunodeficiency virus, visna virus, and the like are known. Lentivirus converts its own RNA into DNA using a reverse transcriptase and inserts the DNA into a host's genomic DNA.

When a lentivirus is used as a viral vector, almost all mammalian cells, including not only dividing cells but also non-dividing cells such as nerve cells and liver cells, stem cells, and the like, for which it is difficult to achieve gene transfer with a general retrovirus vector, can be subjected to gene transfer.

Therefore, a target gene can be easily incorporated into the genome of a target cell by using a lentiviral vector. As the lentiviral vector, an HIV-based vector or an FIV-based vector can be suitably used.

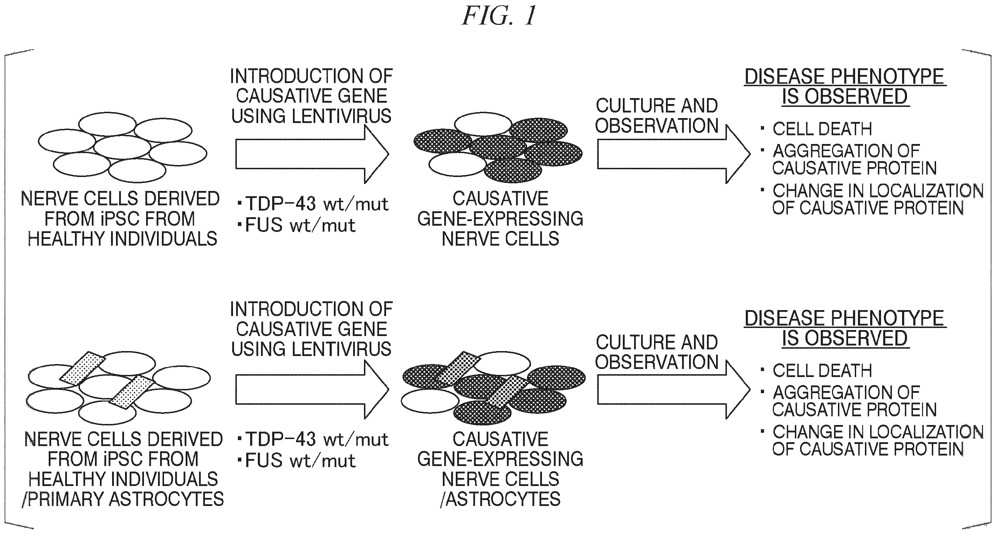

FIG. 1 is a schematic view showing an embodiment of a production method for an ALS model. In the example in the upper row of FIG. 1, nerve cells derived from iPSCs of healthy individuals are single-cultured (the nerve cells are cultured alone), and in the example in the lower row of FIG. 1, nerve cells derived from iPSCs of healthy individuals and astrocytes are co-cultured. In the example in the lower row of FIG. 1, primary cultured cells are used as the astrocytes.

Subsequently, a disease-causative gene reported in ALS patients is subjected to gene transfer using a lentiviral vector. In the example of FIG. 1, wild-type or mutant TDP-43 gene or wild-type or mutant FUS gene is introduced as a causative gene of ALS. As a result, the causative gene of ALS is incorporated into the genome of nerve cells and is stably and forcibly expressed. Here, in a case where nerve cells are co-cultured with astrocytes, the causative gene of ALS is also incorporated into the genome of the astrocytes and is stably and forcibly expressed. These cells can be cultured for a long period of time, for example, 4 weeks or more, and the disease condition phenotype of ALS can be observed.

It is known that the disease condition phenotype of ALS is induced by exposure to various stress conditions. Examples of the stress conditions include inhibition of transcription, and energy shortage. For example, in Oiwa K., et al. (2023), it is described that various stresses induce a change in the localization of TDP-43 from the nucleus to the cytoplasm, and aggregation of TDP-43 protein. The production method of the present embodiment may or may not further include a step of exposing the ALS model cells to such stress conditions.

### [Screening method for prophylactic or therapeutic drug for ALS]

According to an embodiment, the present invention provides a screening method for a prophylactic or therapeutic drug for ALS, the method including: a step of culturing ALS model cells in the presence of a test substance; and a step of evaluating a phenotype of the ALS model cells, in which the phenotype of the ALS model cells being closer to a wild-type phenotype as compared with a phenotype in the absence of the test substance, indicates that the test substance is a prophylactic or therapeutic drug for ALS.

The screening method of the present embodiment is a screening method for evaluating a test substance using the above-described ALS model cells. Since the above-mentioned ALS model cells can be conveniently produced and can reproduce the disease condition phenotype of ALS, a prophylactic drug or therapeutic drug for ALS can be efficiently screened by the screening method of the present embodiment.

The test substance is not particularly limited, and examples thereof include a natural compound library, a synthetic compound library, an existing drug library, and a metabolite library.

With regard to the screening method of the present embodiment, examples of the phenotype of the ALS model cells include the same disease condition phenotype of ALS as described above, and examples thereof include localization of the TDP-43 protein, which is normally localized in the nucleus, in the cytoplasm; aggregation of the TDP-43 protein; localization of the FUS protein, which is normally localized in the nucleus, in the cytoplasm; aggregation of the FUS protein; a decrease in the number of nerve cells after the lapse of a long period of time (for example, 4 weeks) from the expression of the causative gene of ALS; retraction of neurites; and formation of stress granules.

Examples of exhibiting a phenotype closer to the wild-type phenotype compared with the phenotype in the absence of a test substance include an improvement in the proportion of localization of the TDP-43 protein in the nucleus, a reduction in the aggregation of the TDP-43 protein, an improvement in the proportion of the localization of the FUS protein in the nucleus, a reduction in the aggregation of the FUS protein, a reduction in the decrease in the number of nerve cells (cell death), a reduction in the retraction of neurites, and an increase in the formation of stress granules, all in the presence of the test substance, as compared with the phenotype in the absence of the test substance.

### EXAMPLES

Next, the present invention will be described in more detail by way of Examples; however, the present invention is not intended to be limited to the following Examples.

### [Experimental Example 1]

Nerve cells derived from iPSCs from healthy individuals were co-cultured with astrocytes, a disease-causative gene reported in ALS patients was subjected to gene transfer using a lentivirus and forcibly expressed, and the disease phenotype was observed. In the present Experimental Example, as a lentivirus, a SIN-type (self-inactivating type) lentivirus produced by co-transfecting a pLVSIN lentiviral vector plasmid (TaKaRa) and a Packaging Mix (TaKaRa) into Lenti-X 293T cells (TaKaRa) was used, and the wild-type human TDP-43 gene was forcibly expressed downstream of the EF1α promoter.

Human iPSC-derived nerve cells (Excitatory Neurons, Elixirgen Scientific, Inc.) and human-derived primary cultured astrocytes (ScienCell Research Laboratories, Inc.) were seeded on a 384-well plate such that the number of cells per well was 10,000 for the nerve cells and 2,500 for the astrocytes, and the cells were co-cultured. As the culture medium, Neurobasal Plus (Thermo Fisher Scientific, Inc.) to which B-27 Plus Supplement (Thermo Fisher Scientific, Inc.), Glutamax (Thermo Fisher Scientific, Inc.), 200 µM ascorbic acid (FUJIFILM Wako Pure Chemical Corporation), and 10% Neuron Culture medium (FUJIFILM Wako Pure Chemical Corporation) were added was used. Lentivirus was added to the culture medium to infect the nerve cells and the astrocytes, wild-type human TDP-43 gene was introduced into the cells by gene transfer to be forcibly expressed, and the cells were cultured for 6 weeks.

Thereafter, the cells were fixed with paraformaldehyde, and TDP-43 protein was detected by immunochemical staining. A tag was added to the forcibly expressed TDP-43 protein, and the protein was detected using an anti-tag antibody. A FLAG tag was used as the tag. In this manner, the endogenous TDP-43 protein is not detected, and only the TDP-43 protein produced by gene transfer can be detected. Furthermore, the nuclei were stained with 4',6-diamidino-2-phenylindole (DAPI).

FIG. 2 is fluorescence microscopic images showing the results of immunochemical staining. In FIG. 2, "Nucleus" indicates a result of detecting the nucleus, "TDP43 WT" indicates a result of detecting wild-type TDP-43, and "Merge" indicates a result of superimposing these images.

As a result, as indicated by the arrowheads in FIG. 2, a state in which TDP-43, which is normally localized in the nucleus, was localized in the cytoplasm was observed. This result shows that the disease condition phenotype of ALS can be reproduced by the present Experimental Example.

### [Experimental Example 2]

Nerve cells derived from iPSCs from healthy individuals were co-cultured with astrocytes, a disease-causative gene reported in ALS patients was subjected to gene transfer using a lentivirus and forcibly expressed, and the disease phenotype was observed. In the present Experimental Example, as a lentivirus, a SIN-type (self-inactivating type) lentivirus produced by co-transfecting a pLVSIN lentiviral vector plasmid (TaKaRa) and a Packaging Mix (TaKaRa) into Lenti-X 293T cells (TaKaRa) was used, and a mutant human TDP-43 (A382T) gene was forcibly expressed downstream of the EF1α promoter.

The same operation as in Experimental Example 1 was carried out, except that the mutant human TDP-43 (A382T) gene was forcibly expressed. A tag was added to the forcibly expressed TDP-43 (A382T) protein, and the protein was detected using an anti-tag antibody. A FLAG tag was used as the tag.

FIG. 3 is fluorescence microscopic images showing the results of immunochemical staining. In FIG. 3, "Nucleus" indicates a result of detecting the nucleus, "TDP43 A382T" indicates a result of detecting mutant TDP-43 (A382T), and "Merge" indicates a result of superimposing these images.

As a result, as indicated by the arrowheads in FIG. 3, a state in which TDP-43, which is normally localized in the nucleus, was localized in the cytoplasm was observed. This result further supports that the disease condition phenotype of ALS can be reproduced by the present Experimental Example.

The following Table 1 shows the results of calculating the proportions of cells in which a change in the localization of TDP-43 from the nucleus to the cytoplasm occurred in Experimental Example 1 and Experimental Example 2. As shown in Table 1, it was verified that the proportion of cells exhibiting the disease condition phenotype of ALS was high.

**[Table 1]**

| | Proportion of cells in which change in localization of TDP-43 from nuclei to cytoplasm has occurred |
|---|---|
| Control (No forced expression) | 9.9% |
| Forced expression of TDP-43 wt (Experimental Example 1) | 33.7% |
| Forced expression of TDP-43 A382T (Experimental Example 2) | 38.0% |

### [Experimental Example 3]

The same experiments as in Experimental Example 1 and Experimental Example 2 were carried out under stress conditions, and the disease condition phenotype of ALS was observed. As the stress conditions, inhibition of transcription was induced by addition of actinomycin D. Specifically, similarly to Experimental Example 1 and Experimental Example 2, nerve cells derived from iPSCs from healthy individuals were co-cultured with astrocytes, wild-type human TDP-43 gene or mutant human TDP-43 (A382T) gene was forcibly expressed using a lentivirus, and the cells were cultured for 6 weeks. Thereafter, 5 µg/mL of actinomycin D was added to the culture medium, the cells were cultured for 3 hours, and the localization of TDP-43 was observed by immunochemical staining.

The following Table 2 shows the results of calculating the proportions of cells in which a change in the localization of TDP-43 from the nucleus to the cytoplasm occurred. As shown in Table 2, under the stress conditions, cells exhibiting the disease condition phenotype of ALS were observed even in a case where the causative gene of ALS was not forcibly expressed.

**[Table 2]**

| | Proportion of cells in which change in localization of TDP-43 from nuclei to cytoplasm has occurred |
|---|---|
| Stress conditions (No forced expression) | 34.3% |
| Stress conditions (Forced expression of TDP-43 wt) | 53.7% |
| Stress conditions (Forced expression of TDP-43 A382T) | 59.1% |

In order to analyze the disease mechanism, it is considered important that the disease condition phenotype of ALS is reproduced by forced expression of the causative gene of ALS. For this reason, it is considered that an ALS model based on forced expression of the causative gene of ALS is a more useful model for drug discovery research than an ALS model based on the application of stress.

As shown in Table 1 and Table 2, a significant difference was not recognized between the proportion of cells in which a change in the localization occurred due to the forced expression of the causative gene of ALS and the proportion of cells in which a change in the localization occurred due to the application of stress. However, from the viewpoint of reproduction of the disease condition phenotype of ALS, it is considered that an ALS model based on forced expression of the causative gene of ALS is preferred.

### [Experimental Example 4]

Nerve cells derived from iPSCs from healthy individuals were cultured, a disease-causative gene reported in ALS patients was subjected to gene transfer using a lentivirus and forcibly expressed, and the disease phenotype was observed. In the present Experimental Example, as a lentivirus, a SIN-type (self-inactivating type) lentivirus produced by co-transfecting a pLVSIN lentiviral vector plasmid (TaKaRa) and a Packaging Mix (TaKaRa) into Lenti-X 293T cells (TaKaRa) was used, and wild-type human TDP-43 gene and mutant human TDP-43 (A382T) gene were each forcibly expressed downstream of the EF1α promoter.

Human iPSC-derived nerve cells (Excitatory Neurons, Elixirgen Scientific, Inc.) were seeded on a 384-well plate such that the number of cells per well was 1 × 10⁴. As the culture medium, Neurobasal Plus (Thermo Fisher Scientific, Inc.) to which B-27 Plus Supplement (Thermo Fisher Scientific, Inc.), Glutamax (Thermo Fisher Scientific, Inc.), 200 µM ascorbic acid (FUJIFILM Wako Pure Chemical Corporation), and 10% Neuron Culture medium (FUJIFILM Wako Pure Chemical Corporation) were added was used. Lentivirus was added to the culture medium to infect the nerve cells, wild-type human TDP-43 gene and mutant human TDP-43 (A382T) gene were each introduced into the cells by gene transfer to be forcibly expressed, and the cells were cultured for 6 weeks.

FIG. 4 is a graph showing results of measuring changes over time in the number of cells of nerve cells after gene transfer. The axis of ordinate in the graph represents the number of cells observed within one visual field in a microscope observation at a magnification of 20 times, and the axis of abscissa represents the culture time (weeks) after gene transfer. As a result, even in a case where either the wild-type human TDP-43 gene or the mutant human TDP-43 (A382T) gene was forcibly expressed, a significant decrease in the number of cells was observed 4 weeks after the start of the forced expression. This result further supports that the disease condition phenotype of ALS can be reproduced by the present Experimental Example.

### [Experimental Example 5]

Nerve cells derived from iPSCs from healthy individuals were cultured, a disease-causative gene reported in ALS patients was subjected to gene transfer using a lentivirus and forcibly expressed, and the disease phenotype was observed. In the present Experimental Example, as a lentivirus, a SIN-type (self-inactivating type) lentivirus produced by co-transfecting a pLVSIN lentiviral vector plasmid (TaKaRa) and a Packaging Mix (TaKaRa) into Lenti-X 293T cells (TaKaRa) was used, and wild-type human FUS gene was forcibly expressed downstream of the EF1α promoter.

Human iPSC-derived nerve cells (Excitatory Neurons, Elixirgen Scientific, Inc.) were seeded on a 384-well plate such that the number of cells per well was 1 × 10⁴. As the culture medium, Neurobasal Plus (Thermo Fisher Scientific, Inc.) to which B-27 Plus Supplement (Thermo Fisher Scientific, Inc.), Glutamax (Thermo Fisher Scientific, Inc.), 200 µM ascorbic acid (FUJIFILM Wako Pure Chemical Corporation), and 10% Neuron Culture medium (FUJIFILM Wako Pure Chemical Corporation) were added was used. Lentivirus was added to the culture medium to infect the nerve cells, wild-type human FUS gene was introduced into the cells by gene transfer to be forcibly expressed, and the cells were cultured for 6 weeks.

Thereafter, the cells were fixed with paraformaldehyde, and FUS protein was detected by immunochemical staining. A tag was added to the forcibly expressed FUS protein, and only the FUS protein produced by gene transfer could be detected using an anti-tag antibody. A FLAG tag was used as the tag. Furthermore, both the endogenous FUS protein and the forcibly expressed FUS protein were detected by performing immunostaining using an anti-FUS antibody. In addition, the nuclei were stained with DAPI.

FIG. 5 is fluorescence microscopic images showing the results of immunochemical staining. In FIG. 5, "FUS" indicates a result of performing detection with an anti-FUS antibody, "FUS WT" indicates a result of performing detection with an anti-tag antibody, "Nucleus" indicates a result of detecting the nucleus, and "Merge" indicates a result of superimposing these images.

As a result, as indicated by the arrowheads in FIG. 5, a state in which FUS, which is normally localized in the nucleus, was localized in the cytoplasm was observed. This result further supports that the disease condition phenotype of ALS can be reproduced by the present Experimental Example.

### [Experimental Example 6]

Nerve cells derived from iPSCs from healthy individuals were cultured, a disease-causative gene reported in ALS patients was subjected to gene transfer using a lentivirus and forcibly expressed, and the disease phenotype was observed. In the present Experimental Example, as a lentivirus, a SIN-type (self-inactivating type) lentivirus produced by co-transfecting a pLVSIN lentiviral vector plasmid (TaKaRa) and a Packaging Mix (TaKaRa) into Lenti-X 293T cells (TaKaRa) was used, and mutant human FUS (R521C) gene was forcibly expressed downstream of the EF1α promoter.

The same operation as in Experimental Example 4 was carried out, except that the mutant human FUS (R521C) gene was forcibly expressed. A tag was added to the forcibly expressed FUS (R521C) protein, and the protein was detected using an anti-tag antibody. A FLAG tag was used as the tag.

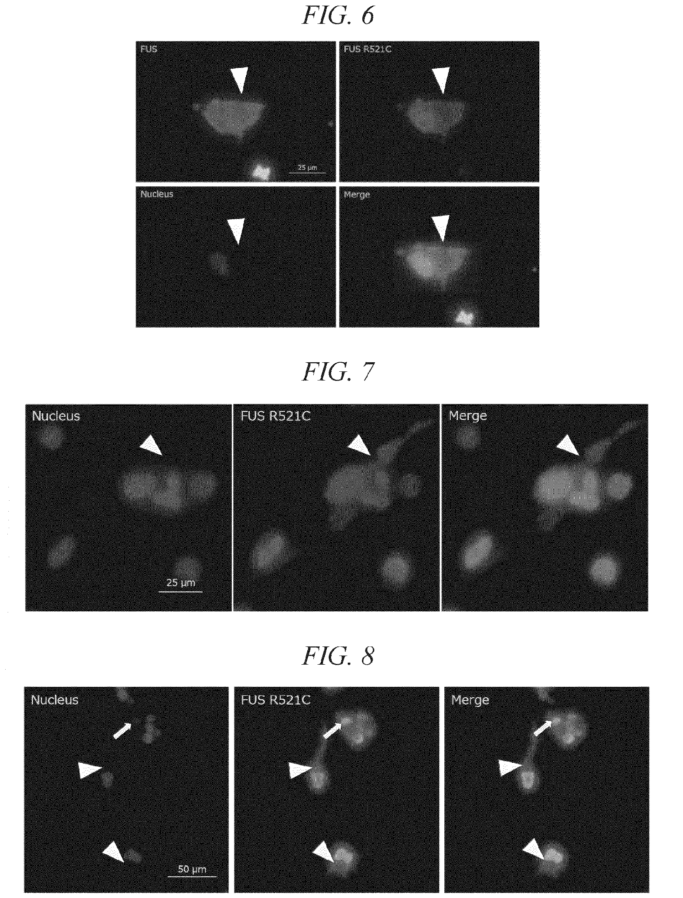

FIG. 6 is fluorescence microscopic images showing the results of immunochemical staining. In FIG. 6, "FUS" indicates a result of performing detection with an anti-FUS antibody, "FUS R521C" indicates a result of performing detection with an anti-tag antibody, "Nucleus" indicates a result of detecting the nucleus, and "Merge" indicates a result of superimposing these images.

As a result, as indicated by the arrowheads in FIG. 6, a state in which FUS, which is normally localized in the nucleus, was localized in the cytoplasm was observed. This result further supports that the disease condition phenotype of ALS can be reproduced by the present Experimental Example.

### [Experimental Example 7]

The same experiment as in Experimental Example 6 was carried out, except that the nerve cells were co-cultured with astrocytes. Human iPSC-derived nerve cells (Excitatory Neurons, Elixirgen Scientific, Inc.) and human-derived primary cultured astrocytes (ScienCell Research Laboratories, Inc.) were seeded on a 384-well plate such that the number of cells per well was 10,000 for the nerve cells and 2,500 for the astrocytes, and the cells were co-cultured. As the culture medium, Neurobasal Plus (Thermo Fisher Scientific, Inc.) to which B-27 Plus Supplement (Thermo Fisher Scientific, Inc.), Glutamax (Thermo Fisher Scientific, Inc.), 200 µM ascorbic acid (FUJIFILM Wako Pure Chemical Corporation), and 10% Neuron Culture medium (FUJIFILM Wako Pure Chemical Corporation) were added was used. Lentivirus was added to the culture medium to infect the nerve cells and the astrocytes, mutant human FUS (R521C) gene was forcibly expressed, and the cells were cultured for 6 weeks.

Thereafter, the cells were fixed with paraformaldehyde, and mutant FUS (R521C) protein was detected by immunochemical staining. In addition, the nuclei were stained with DAPI.

FIG. 7 is fluorescence microscopic images showing the results of immunochemical staining. In FIG. 7, "Nucleus" indicates a result of detecting the nucleus, "FUS R521C" indicates a result of detecting mutant FUS (R521C), and "Merge" indicates a result of superimposing these images.

As a result, as indicated by the arrowheads in FIG. 7, a state in which FUS, which is normally localized in the nucleus, was localized in the cytoplasm was observed. This result further supports that the disease condition phenotype of ALS can be reproduced by the present Experimental Example.

### [Experimental Example 8]

The same experiment as in Experimental Example 6 was carried out again. Nerve cells derived from iPSCs from healthy individuals were cultured, mutant human FUS (R521C) gene was forcibly expressed downstream of the EF1α promoter using a lentivirus (a SIN-type (self-inactivating type) lentivirus produced by co-transfecting a pLVSIN lentiviral vector plasmid (TaKaRa) and a Packaging Mix (TaKaRa) into Lenti-X 293T cells (TaKaRa)), and the cells were cultured for 6 weeks.

Thereafter, the cells were fixed with paraformaldehyde, and mutant FUS (R521C) protein was detected by immunochemical staining. In addition, the nuclei were stained with DAPI.

FIG. 8 is fluorescence microscopic images showing the results of immunochemical staining. In FIG. 8, "Nucleus" indicates a result of detecting the nucleus, "FUS R521C" indicates a result of detecting mutant FUS (R521C) protein, and "Merge" indicates a result of superimposing these images.

As a result, as indicated by the arrowheads in FIG. 8, a state in which FUS, which is normally localized in the nucleus, was localized in the cytoplasm was observed. Furthermore, as indicated by the arrowheads in FIG. 8, a state in which mutant FUS (R521C) protein formed aggregates was observed. This result further supports that the disease condition phenotype of ALS can be reproduced by the present Experimental Example.

The present invention includes the following embodiments.
[1] A nerve cell having: a causative gene of amyotrophic lateral sclerosis (ALS) incorporated into a genome of the nerve cell in an acquired manner, the causative gene being operably linked to a promoter for a eukaryotic cell.
[2] The nerve cell according to [1], in which the nerve cell is a nerve cell that has been induced to be differentiated from a pluripotent stem cell derived from a healthy individual.
[3] The nerve cell according to [1] or [2], in which the causative gene of ALS is a TAR DNA binding protein-43 (TDP-43) gene, a fused in sarcoma (FUS) gene, or a Superoxide Dismutase 1 (SOD1) gene, each being a wild-type gene or a mutant gene.
[4] The nerve cell according to any one of [1] to [3], in which the nerve cell exhibits a disease condition phenotype of ALS, and the disease condition phenotype of ALS is cell death, or a change in localization of a TDP-43 protein, a FUS protein, or a SOD1 protein, or aggregation of a TDP-43 protein, a FUS protein, or a SOD1 protein.
[5] A co-culture product including: the nerve cell according to any one of [1] to [4]; and an astrocyte in which a causative gene of ALS operably linked to a promoter for a eukaryotic cell is incorporated into a genome of the astrocyte.
[6] The co-culture product according to [5], in which the causative gene of ALS incorporated into the genome of the astrocyte is the same gene as the causative gene of ALS incorporated into the genome of the nerve cell.
[7] A production method for ALS model cells, the method including: a step of introducing a causative gene of ALS operably linked to a promoter for a eukaryotic cell, into a genome of a nerve cell that has been induced to be differentiated from a pluripotent stem cell, or into a genome of a co-culture product of the nerve cell and an astrocyte.
[8] The production method according to [7], in which the step of introducing the causative gene of ALS is carried out using a lentiviral vector.
[9] The production method according to [7] or [8], further including a step of culturing the cells for 4 weeks or more, after the step of introducing the causative gene of ALS.
[10] The production method according to any one of [7] to [9], in which the nerve cell is a nerve cell that has been induced to be differentiated from a pluripotent stem cell derived from a healthy individual.
[11] The production method according to any one of [7] to [10], in which the causative gene of ALS is a TDP-43 gene, a FUS gene, or a SOD1 gene, each being a wild-type gene or a mutant gene.
[12] A screening method for a prophylactic or therapeutic drug for ALS, the method including: a step of culturing ALS model cells in presence of a test substance; and a step of evaluating a phenotype of the ALS model cells, in which the phenotype of the ALS model cells being closer to a wild-type phenotype as compared with a phenotype in absence of the test substance, implies that the test substance is a prophylactic or therapeutic drug for ALS.
[13] The screening method according to [12], in which the phenotype of the ALS model cells is cell death, or a change in localization of a TDP-43 protein, a FUS protein, or a SOD1 protein, or aggregation of a TDP-43 protein, a FUS protein, or a SOD1 protein.

### [Related Art Document]

### [Patent Document]

Patent Document 1: Japanese Unexamined Patent Application, First Publication No. 2023-140198

### [Non-Patent Documents]

[Non-Patent Document 1] Oiwa K., et al., Monomerization of TDP-43 is a key determinant for inducing TDP-43 pathology in amyotrophic lateral sclerosis, Sci Adv., 9, eadf6895, 2023.
[Non-Patent Document 2] Vogt M.A. et al., TDP-43 induces p53-mediated cell death of cortical progenitors and immature neurons, Sci Rep., 8, 8097, 2018.
[Non-Patent Document 3] Zhang Z., et al., Downregulation of MicroRNA-9 in iPSC-Derived Neurons of FTD/ALS Patients with TDP-43 Mutations, PLOS ONE, 8, 10, e76055, 2013.
[Non-Patent Document 4] Fujimori K., et al., Modeling sporadic ALS in iPSC-derived motor neurons identifies a potential therapeutic agent, Nature Medicine, 24, 1579-1589, 2018.

While preferred embodiments of the invention have been described and illustrated above, it should be understood that these are exemplary of the invention and are not to be considered as limiting. Additions, omissions, substitutions, and other modifications can be made without departing from the scope of the invention. Accordingly, the invention is not to be considered as being limited by the foregoing description and is only limited by the scope of the appended claims.

## Claims

1. A nerve cell comprising:
a causative gene of amyotrophic lateral sclerosis (ALS) incorporated into a genome of the nerve cell in an acquired manner, the causative gene being operably linked to a promoter for a eukaryotic cell.

2. The nerve cell according to claim 1,
wherein the nerve cell is a nerve cell that has been induced to be differentiated from a pluripotent stem cell derived from a healthy individual.

3. The nerve cell according to claim 1 or 2,
wherein the causative gene of ALS is a TAR DNA binding a protein-43 (TDP-43) gene, a fused in sarcoma (FUS) gene, or a Superoxide Dismutase 1 (SOD1) gene, each being a wild-type gene or a mutant gene.

4. The nerve cell according to claim 1 or 2,
wherein the nerve cell exhibits a disease condition phenotype of ALS, and the disease condition phenotype of ALS is cell death, or a change in localization of a TDP-43 protein, a FUS protein, or a SOD1 protein, or an aggregation of a TDP-43 protein, a FUS protein, or a SOD1 protein.

5. A co-culture product comprising:
the nerve cell according to claim 1 or 2; and
an astrocyte in which a causative gene of ALS operably linked to a promoter for a eukaryotic cell is incorporated into a genome of the astrocyte.

6. The co-culture product according to claim 5,
wherein the causative gene of ALS incorporated into the genome of the astrocyte is the same gene as the causative gene of ALS incorporated into the genome of the nerve cell.

7. A production method for ALS model cells, the method comprising:
a step of introducing a causative gene of ALS operably linked to a promoter for a eukaryotic cell, into a genome of a nerve cell that has been induced to be differentiated from a pluripotent stem cell, or into a genome of a co-culture product of the nerve cell and an astrocyte.

8. The production method according to claim 7,
wherein the step of introducing the causative gene of ALS is carried out using a lentiviral vector.

9. The production method according to claim 7 or 8, further comprising:
a step of culturing the cells for 4 weeks or more, after the step of introducing the causative gene of ALS.

10. The production method according to claim 7 or 8,
wherein the nerve cell is a nerve cell that has been induced to be differentiated from a pluripotent stem cell derived from a healthy individual.

11. The production method according to claim 7 or 8,
wherein the causative gene of ALS is a TDP-43 gene, a FUS gene, or a SOD1 gene, each being a wild-type gene or a mutant gene.

12. A screening method for a prophylactic or therapeutic drug for ALS, the method comprising:
a step of culturing ALS model cells in presence of a test substance; and
a step of evaluating a phenotype of the ALS model cells,
wherein the phenotype of the ALS model cells being closer to a wild-type phenotype as compared with a phenotype in absence of the test substance, implies that the test substance is a prophylactic or therapeutic drug for ALS.

13. The screening method according to claim 12,
wherein the phenotype of the ALS model cells is cell death, or a change in localization of a TDP-43 protein, a FUS protein, or a SOD1 protein, or an aggregation of a TDP-43 protein, a FUS protein, or a SOD1 protein.
